# EUROPEAN PATENT SPECIFICATION

(11) **EP 4 004 547 B1**
(45) Date of publication and mention of the grant of the patent: **11.10.2023**
(21) Application number: 21717462.2
(22) Date of filing: 13.04.2021
(51) Int. Cl.: G01N 33/569

(54) **METHODS FOR DETECTING THE PRESENCE OF CORONAVIRUS-SPECIFIC ANTIBODIES IN A SUBJECT**
VERFAHREN ZUR DETEKTION DER ANWESENHEIT VON CORONAVIRUS-SPEZIFISCHEN ANTIKÖRPERN IN EINEM SUBJEKT
PROCÉDÉS DE DÉTECTION DE LA PRÉSENCE D'ANTICORPS SPÉCIFIQUES DE CORONAVIRUS CHEZ UN SUJET

(30) Priority: 14.04.2020 EP 20315157; 09.06.2020 EP 20305614
(43) Date of publication of application: 01.06.2022
(60) Divisional application: 23194718.5
(73) Proprietor: (INSERM) Institut National de la Santé et de la Recherche Médicale, 75013 Paris (FR); Université de Rouen Normandie, 76130 Mont-Saint-Aignan (FR); Centre Hospitalier Universitaire de Rouen, 76000 Rouen (FR)
(72) Inventor: BOYER, Olivier, 76000 Rouen (FR); DROUOT, Laurent, 76000 Rouen (FR)
(74) Representative: Santarelli
(86) International application number: PCT/EP2021/059583
(87) International publication number: WO 2021/209463

(56) References cited:
- WO-A2-2005/012337
- US-A1- 2005 112 559
- US-B1- 7 220 852
- Xuefei Cai ET AL: "A Peptide-based Magnetic Chemiluminescence Enzyme Immunoassay for Serological Diagnosis of Corona Virus Disease 2019 (COVID-19)", medRxiv, 25 February 2020 (2020-02-25), XP055742472, DOI: 10.1101/2020.02.22.20026617 Retrieved from the Internet: URL:https://www.medrxiv.org/content/10.110 1/2020.02.22.20026617v1.full.pdf [retrieved on 2020-10-21]
- Fatima Amanat ET AL: "A serological assay to detect SARS-CoV-2 seroconversion in humans", medRxiv, 18 March 2020 (2020-03-18), XP055742527, DOI: 10.1101/2020.03.17.20037713 Retrieved from the Internet: URL:https://www.medrxiv.org/content/10.110 1/2020.03.17.20037713v1.full.pdf [retrieved on 2020-10-21]

## Description

### FIELD OF THE INVENTION:

The present invention is in the field of medicine in particular immunology and virology.

### BACKGROUND OF THE INVENTION:

*Coronaviridae* is a family of enveloped, positive-sense, single-stranded RNA viruses. The viral genome is 26-32 kilobases in length. The particles are typically decorated with large (~20 nm), club- or petal-shaped surface projections (the "peplomers" or "spikes"), which in electron micrographs of spherical particles create an image reminiscent of the solar corona. In late December 2019, a new betacoronavirus SARS-CoV-2 has emerged in Wuhan China [1-3]. The World Health Organization has named the severe pneumonia caused by this new coronavirus COVID-19 (for Corona Virus Disease 2019, WHO, 2020). Since its emergence, the SARS-CoV-2 has spread to 159 countries across the five continents causing, at the time of the writing, about 213,254 human infections with 81,238 cases in China (ECDC, March 19 2020). Europe has recently become the epicenter of COVID-19 epidemics with 82,869 confirmed cases; the majority of them being reported in Italy with 35,713 cases and 2978 deaths. In France, the number of confirmed cases is increasing with about 10,995 and 372 deaths on mid-march 2019 (Santé Publique France). To fight against the COVID-19 pandemic in a long term, in addition to the containment measures implemented in many countries, reliable diagnostic methods are highly desirable.

The reference standard of molecular test for diagnosis of COVID-19 is reverse transcription-polymerase chain reaction (RT-PCR), which detects viral RNA using nasopharyngeal swabs or other upper respiratory tract specimens. Therefore, RT-PCR remains the primary method of diagnosing SARS-CoV-2 despite limitations including false-negative or false-positive results due to the technique itself, insufficient amount of material at the site of sample collection, or inappropriate time of sampling. Serological tests are essential complements to molecular tests because they can identify individuals with SARS-CoV-2 at a distance from infection, when RT-PCR has become negative or was inconclusive.

Besides diagnosis, serological tests are useful for epidemiological purposes, vaccination research, and, possibly, for assessment of the level of protection toward reinfection. Serological assays evaluate the humoral immune response to nucleocapsid (N) or Spike (S) proteins as they have been shown to be the most immunogenic proteins among coronaviruses (Meyer et al., 2014). In particular, the development and availability of tests for the detection and quantification of anti-SARS-CoV-2 antibodies in subjects with COVID-19 is of strong diagnostic interest. On an individual basis, they would potentially provide information on the state of protection against reinfection. They would also make it possible to determine a posteriori the level of exposure of the population to the virus, thus constituting an element likely to guide a strategy of containment or lifting of containment.

Serological tests include lateral flow immunoassays (LFIAs), chemiluminescent assays (CLIAs), bead-based assays, immunometric luminescence, electrochemiluminescence immunoassays, or enzyme-linked immunosorbent assays (ELISA). Tests typically detect the presence of antibodies (Abs) against the S protein or its domains (S 1, S2, or RBD) and/or the N protein. The sensitivity of SARS-CoV-2 immunoassays may vary widely according to the time when serum samples were collected, with a higher sensitivity for CLIAs and ELISAs than for LFIAs, whereas the specificity of the different tests is typically higher than 95% (Lisboa Bastos et al., 2020).

To date, in addition to non-quantitative rapid tests, only tests based on the ELISA, CLIA or LFIA method have been reported.

In a recent study, Cai et al. developed a peptide-based luminescent immunoassay to detect anti-protein S IgG and IgM in the 276 sera from confirmed patients. They evaluated the sensitivity of the test at 71.4 % , 57.2% and 81,5% for IgG, IgM and IgM+G respectively.

In another recent study, Guo et al. analyzed the humoral response (IgA, IgM and IgG) in 82 confirmed patients and 58 subjects suspected of COVID-19 (qPCR negative but suggestive clinical signs). Using an ELISA test using recombinant N capsid protein as antigen, they detected anti-nucleocapsid IgM and IgA 5 days after the onset of the first symptoms and 14 days for IgG. The positive serum levels were 85, 93 and 78% respectively. The anti-nucleocapsid N IgM positivity between positive and probably infected subjects was 76% and 93% respectively. The authors also showed that a serological test such as this one allowed an earlier diagnosis of the disease compared to quantitative PCR which relies on the detection of the viral genome with a possibly less sensitive technique. Moreover, the combination of the two techniques significantly increased the detection rate of positive sera to 98.6% (Guo, 2020).

Xiang and colleagues using an ELISA from Zhu Hai Liv Zon Diagnostics evaluated the sensitivity of the test at 44, 82 and 87% for IgM, IgG and IgM+G respectively for a specificity of 100%. Reproducibility was 64, 89 and 92%, respectively. The combined use of an IgM ELISA and an IgG ELISA (here referred to as IgM+G ELISA) appears to be even more sensitive than qPCR (87% vs. 52%). However, the antigen used in this study is not known.

In another study, Xia et al. did similar work with an ELISA using an unspecified antigen and came to the same conclusion. The sensitivity of the combined IgM+G ELISA is superior to that of single class ELISAs and to that of the qPCR.

EUROIMMUN develops ELISAs using different coronavirus proteins including the Spike protein of SARS-CoV-2 produced in HEK293T cells. Using this test under development, Okba and collaborators have demonstrated cross-reactivity between different coronaviruses.

Liu and colleagues using an anti-nucleocapsid and S-protein ELISA from Lizhu (Zhuhai, China), detected with high sensitivity anti-SARS-Cov-2 IgM and IgG in 214 patients. The IgM+G combination offers higher sensitivity than ELISAs detecting a single immunoglobulin class (IgM or IgG). Finally, the anti-protein S ELISA is more sensitive than the anti-nucleocapsid ELISA. Finally, Liu and colleagues from the same bridging laboratory concluded with an anti-nucleocapsid ELISA that the sensitivity of this test was also higher than that of the qPCR.

The ELISA method has the advantage that it can be used in many diagnostic laboratories. However, the ELISA method requires a large amount of antigenic protein per measuring point, which has an impact on the cost price. It does not allow the simultaneous detection of several antibodies in the same well, which would be useful in the context of an antiviral response against several antigens.

The Adressable Laser Beads ImmunoAssay (ALBIA) method based on the Luminex^{™} Technology is based on the principle of flow cytometry. It combines the use of fluorescent polystyrene microbeads on which the target antigens are fixed and a double reading by two lasers detecting the signals emitted by the microbeads and a coupled secondary antibody. Using a panel of beads coupled to different antigens and containing a different ratio of red and orange fluorescence, it is possible to multiplex the assays and detect several antibodies in the same well. However, said method has not yet been investigated for diagnosis of coronavirus infection.

### SUMMARY OF THE INVENTION:

As defined by the claims, the present invention relates to methods for detecting the presence of SARS-Cov-2-specific antibodies in a subject.

### DETAILED DESCRIPTION OF THE INVENTION:

Here, the inventors have developed a multiplex addressable laser bead immunoassay (ALBIA) to detect and quantify IgG Abs against the Spike S1 domain and nucleocapsid N of SARS-Cov-2, and a monoplex ALBIA to assay for anti-S1 IgM Abs. Recombinant S1 and N proteins were bound to fluorescent beads (ALBIA-IgG-S 1/N). Abs were revealed using class-specific anti-human Ig Abs. The performances of the test were analyzed on 575 serum samples including 192 from SARS-CoV-2 polymerase chain reaction-confirmed patients, 13 from seasonal coronaviruses, 70 from different inflammatory/autoimmune diseases, and 300 from healthy donors. Anti-S1 IgM were detected by monoplex ALBIA-IgM-S1. Multiplex ALBIA-IgG-S1/N was effective in detecting and quantifying anti-SARS-CoV-2 IgG Abs. Two weeks after first symptoms, sensitivity and specificity were 97.7 and 98.0% (anti-S1), and 100 and 98.7% (anti-N), respectively.

Accordingly, the first object of the present invention relates to a method for detecting the presence of SARS-Cov-2-specific antibodies in a subject, comprising the steps of:
a) placing a sample obtained from the subject in a single assay receptacle, in the presence of a plurality of particles belonging to at least two different groups, one of the groups being conjugated to a first coronaviral polypeptide and one other group being conjugated to a second coronaviral polypeptide,
b) incubating the mixture under conditions which allow the formation of immunocomplexes on each group of particles,
c) eliminating from the mixture of step b) the immunoglobulins which have not bound to the particles,
d) incubating the mixture of step c) with at least an anti-human IgG antibody that is coupled to a fluorescent label;
e) eliminating from the mixture of step d) the anti-human IgG not bound to the immunocomplexes formed in step b), and
f) simultaneously detecting, by means of a detector capable of differentiating the at least two groups of particles mentioned above, the immunocomplexes formed in step d) on each particle, whereby the presence or absence of SARS-CoV-2-specific antibodies is revealed and wherein the fluorescence of the fluorescent label is measured to quantify the detected SARS-CoV-2-specific antibodies,
wherein one of the first and second coronaviral polypeptides derives from the SARS-CoV-2 nucleoprotein (N) and the other coronaviral polypeptide derives from the S 1 subunit of the SARS-CoV-2 spike (S) protein.

SARS-CoV-2 is a coronavirus whose genome is plus-stranded RNA of about 27 kb to about 33 kb in length depending on the particular virus. The virion RNA has a cap at the 5' end and a poly A tail at the 3' end. The length of the RNA makes coronaviruses the largest of the RNA virus genomes. In particular, SARS-CoV-2 RNAs encode: (1) an RNA-dependent RNA polymerase; (2) N-protein; (3) three envelope glycoproteins; plus (4) three non-structural proteins. In particular, the SARS-CoV-2 particle comprises at least the four canonical structural proteins E (envelope protein), M (membrane protein), N (nucleocapsid protein), and S (spike protein). The S protein is cleaved into 3 chains: Spike protein S1, Spike protein S2 and Spike protein S2'. Production of the replicase proteins is initiated by the translation of ORFla and ORFlab via a -1 ribosomal frame-shifting mechanism. This mechanism produces two large viral polyproteins, ppla and pplab, that are further processed by two virally encoded cysteine proteases, the papain-like protease (PLpro) and a 3C-like protease (3CLpro), which is sometimes referred to as main protease (Mpro). SARS-CoV-2 is transmitted by aerosols of respiratory secretions.

In some embodiments, the subject can be human or any other animal susceptible to SARS-CoV-2 infection (e.g. domestic animals such as cats and dogs; livestock and farm animals such as horses, cows, pigs, chickens, etc.). In some embodiments, the subject is a non-human animal. In some embodiments, the subject is a farm animal or pet. In some embodiments, the subject is a human. In some embodiments, the subject is a human infant. In some embodiments, the subject is a human child. In some embodiments, the subject is a human adult. In some embodiments, the subject is an elderly human. In some embodiments, the subject is a premature human infant.

In some embodiments, the subject can be symptomatic or asymptomatic. As used herein, the term "**asymptomatic**" refers to a subject who experiences no detectable symptoms for the SARS-CoV-2 infection. As used herein, the term "**symptomatic**" refers to a subject who experiences detectable symptoms of SARS-CoV-2 infection. Symptoms of SARS-CoV-2 infection include: fatigue, anosmia, headache, cough, fever, difficulty to breathe.

As used herein, the term "**sample**" refers to a biological sample obtained for the purpose of in vitro evaluation. Typical biological samples to be used in the method according to the invention are blood samples (e.g. whole blood sample or serum sample). In some embodiments, said biological liquids comprise blood, plasma, serum, saliva and exsudates. Thus, in some embodiments, the sample is chosen from blood samples, plasma samples, saliva samples, exsudate samples and serum samples. Preferably, the sample is a blood sample, a serum sample or a plasma sample.

As used herein, the term "**antibody**", **"immunoglobulins"** or "**Igs**" has its general meaning in the art and relates to proteins of the immunoglobulin superfamily. The immunoglobulins are characterized by a structural domain, i.e., the immunoglobulin domain, having a characteristic immunoglobulin (Ig) fold. The term encompasses secretory immunoglobulins. Immunoglobulins generally comprise several chains, typically two identical heavy chains and two identical light chains which are linked via disulfide bonds. These chains are primarily composed of immunoglobulin domains, including the VL domain (light chain variable domain), the CL domain (light chain constant domain), the VH domain (heavy chain variable domain) and the CH domains (heavy chain constant domains) CH1, optionally a hinge region, CH2, CH3, and optionally CH4. There are five main heavy chain classes (or isotypes) which determine the functional activity of an antibody molecule: mu (µ) for IgM, delta (δ) for IgD, gamma (γ) for IgG, alpha (a) for IgA and epsilon (ε) for IgE. In the context of the invention, IgG specific for S1 and N proteins are detected. Other immunoglobulins which may be detected, in addition to said IgG, are IgM, IgD, IgG, IgA or IgE. As well-known from the skilled person, the IgG isotype encompasses four subclasses: the subclasses lgG1, lgG2, lgG3 and lgG4. The IgA isotype encompasses 2 subclasses: IgA1 and IgA2 immunoglobulins.

As used herein, the term "**particle**" has its general meaning in the art and refers to a particle from 1 nm to 1000 nm, preferably from 100 to 500 nm and even more preferably from 350 to 450nm in size. In some embodiments, the size of the particle is about 400nm. A particle may typically be spherical, though the shape is not limited to that of a sphere and may include other shapes like spheroid, irregular particles, cubes, irregular cubes, and disks. According to the present invention the term "**particle**" is interchangeable with the term "**bead**".

In some embodiments, the particle of the present invention is made of an organic polymer. Organic polymers encompass, but are not limited to, polystyrene, poly(vinyl acetate), poly(methylstyrene), poly(acrylamide), poly(acrylonitrile), poly(vinyl chloride), poly(butyl acrylate), poly(acrylic acid), copolymers of styrene and C 1-C4alkyl (meth)acrylate, copolymers of styrene and acrylamide, copolymers of styrene and acrylonitrile, copolymers of styrene and vinyl acetate, copolymers of acrylamide and C1-C4 alkyl (meth)acrylates, copolymers from acrylonitrile and C1-C4 alkyl (meth)acrylate, copolymers of acrylonitrile and acrylamide, terpolymers from styrene, acrylonitrile and acrylamide, poly(methyl methacrylate), poly(ethyl methacrylate), copolymers styrene/butadiene, styrene/acrylic acid, styrene/vinylpyrrolidone and butadiene/acrylonitrile, or methoxy poly(ethylene glycol)-poly(lactide) copolymer (MPEG-PLA). Polymer particles can be crosslinked or not. For instance, organic particles include, but are not limited to, nylon (for example marketed by ATOCHEM), polyethylene powders (for example marketed by PLAST LABOR), poly-2-alanine powders, polyfluorinated powders such as polytetrafluoroethylene (for example marketed by DUPONT DE NEMOURS), acrylic copolymer powders (for example marketed by DOW CHEMICA), polystyrene powders (for example marketed by PRESPERESE), polyester powders, expanded microspheres in thermoplastic material (for example marketed by EXPANCEL), microballs of silicon resins (for example marketed by TOSHIBA), synthetic hydrophilic polymer powders such as polyacrylates (for example marketed by MATSUMOTO), acrylic polyamides (for example marketed by ORIS), insoluble polyurethanes (for example marketed by TOSHNU), porous microspheres of cellulose, micro- or particles of PTFE (polytetrafluoroethylene).

In some embodiments, the particles are selected to have a variety of properties useful for particular experimental formats. For example, particles can be selected that remain suspended in a solution of desired viscosity or to readily precipitate in a solution of desired viscosity. Particles also can be coded for identification purposes, such as by bar codes, luminescence, fluorescence and the like. A variety of coded particles are well known to those skilled in the art, and include for example, Luminex^{®} and Cyvera^{®} coded particles. With regard to coded particles, each particle can include a unique code, preferably, the coded particles contain a code other than that present in the detectable tag used to detect the presence or amount of modified substrate (e.g., support-bound product portion, free product portion, or modified support-bound substrate). The code can be embedded (for example, within the interior of the particle) or otherwise attached to the particle in a manner that is stable through hybridization and analysis. The code can be provided by any detectable means, such as by holographic encoding, by a fluorescence property, color, shape, size, light emission, quantum dot emission and the like to identify particle and thus the capture probes immobilized thereto. For example, the particles may be encoded using optical, chemical, physical, or electronic tags. Examples of such coding technologies are optical bar codes fluorescent dyes, or other means. One exemplary platform utilizes mixtures of fluorescent dyes impregnated into polymer particles as the means to identify each member of a particle set to which a specific capture probe has been immobilized. Another exemplary platform uses holographic barcodes to identify cylindrical glass particles. For example, Chandler et al. (U.S. Pat. No. 5,981,180) describes a particle-based system in which different particle types are encoded by mixtures of various proportions of two or more fluorescent dyes impregnated into polymer particles. Soini (U.S. Pat. No. 5,028,545) describes a particle-based multiplexed assay system that employs time-resolved fluorescence for particle identification. Fulwyler (U.S. Pat. No. 4,499,052) describes an exemplary method for using particle distinguished by color and/or size. U.S. Patent Publication Nos. 2004-0179267, 2004-0132205, 2004-0130786, 2004-0130761, 2004-0126875, 2004-0125424, and 2004-0075907 describe exemplary particles encoded by holographic barcodes. U.S. Pat. No. 6,916,661 describes polymeric particles (e.g., microparticles) that are associated with particles that have dyes that provide a code for the particles.

As used herein, the term "**antigen**" refers to a substance that can cause the immune system to produce an antibody response against it, and possibly can trigger a biological reaction when an antibody binds to it under the appropriate in vivo conditions. The term antigen as used herein shall refer to a whole target molecule or a fragment of such molecule recognized by an antigen binding site. Specifically, substructures of an antigen, e.g. a polypeptide, generally referred to as "**epitopes**", which are immunologically relevant, may be recognized by an antibody. In particular, an antigen according to the present invention is a SARS-CoV-2 polypeptide such as described herein after and typically includes N and S 1 antigens. Thus, in some embodiments, the antigen of the present invention comprises at least one epitope. Methods for identifying and characterizing epitopes are well known in the art. Typically, said methods include but are not limited to epitope prediction algorithms and MHC associated peptidome identified by mass spectrometry (MS).

As used herein, the terms **"polypeptide", "peptide,"** and **"protein"** are used interchangeably herein to refer to polymers of amino acids of any length. Polypeptides when discussed in the context of the present invention refer to the respective intact polypeptide, or any fragment or genetically engineered derivative thereof, which retains the desired biochemical function and/or conformation of the intact protein.

In the above method, one of the first and second SARS-CoV-2 polypeptides derives from the nucleoprotein (N) protein. In some embodiments not mentioned in the claims of this patent, but still not inconsistent with the claimed subject-matter, this polypeptide has an amino acid sequence having at least 90% of identity with the amino acid sequence as set forth in SEQ ID NO:1.

SEQ ID NO:1 >sp|P0DTC9|NCAP_SARS2 Nucleoprotein OS=Severe acute
respiratory syndrome coronavirus 2 OX=2697049 GN=N PE=1 SV=1
MSDNGPQNQRNAPRITFGGPSDSTGSNQNGERSGARSKQRRPQGLPNNTASWFTALTQHG
KEDLKFPRGQGVPINTNSSPDDQIGYYRRATRRIRGGDGKMKDLSPRWYFYYLGTGPEAG
LPYGANKDGIIWVATEGALNTPKDHIGTRNPANNAAIVLQLPQGTTLPKGFYAEGSRGGS
QASSRSSSRSRNSSRNSTPGSSRGTSPARMAGNGGDAALALLLLDRLNQLESKMSGKGQQ
QQGQTVTKKSAAEASKKPRQKRTATKAYNVTQAFGRRGPEQTQGNFGDQELIRQGTDYKH
WPQIAQFAPSASAFFGMSRIGMEVTPSGTWLTYTGAIKLDDKDPNFKDQVILLNKHIDAY
KTFPPTEPKKDKKKKADETQALPQRQKKQQTVTLLPAADLDDFSKQLQQSMSSADSTQA

In the above method, the other of the first and second SARS-CoV-2, polypeptides derives from the S1 protein. In some embodiments, another SARS-CoV-2 polypeptide derives from the S protein. In some embodiments, another SARS-CoV-2 polypeptide derives from the S2 protein. In some embodiments, another SARS-CoV-2 polypeptide derives from the S2' protein. In some embodiments, not mentioned as such in the claims of this patent, but still not inconsistent with the claimed subject-matter, the SARS-CoV-2 polypeptide has an amino acid sequence having at least 90% of identity with the amino acid sequence as set forth in SEQ ID NO:2. In some embodiments, not mentioned as such in the claims of this patent, but still not inconsistent with the claimed subject-matter, the SARS-CoV-2 polypeptide has an amino acid sequence having at least 90% of identity with the amino acid sequence that ranges from the amino acid residue at position 13 to the amino acid residue at position 685 in SEQ ID NO:2 ("S1 protein"). In some embodiments, not mentioned as such in the claims of this patent, but still not inconsistent with the claimed subject-matter, the SARS-CoV-2 polypeptide has an amino acid sequence having at least 90% of identity with the amino acid sequence that ranges from the amino acid residue at position 686 to the amino acid residue at position 1273 in SEQ ID NO:2 ("S2 protein"). In some embodiments, not mentioned as such in the claims of this patent, but still not inconsistent with the claimed subject-matter, the SARS-CoV-2 polypeptide has an amino acid sequence having at least 90% of identity with the amino acid sequence that ranges from the amino acid residue at position 816 to the amino acid residue at position 1273 in SEQ ID NO:2 ("S2' protein").

SEQ ID NO:2 >sp|P0DTC2|SPIKE_SARS2 Spike glycoprotein OS=Severe acute
respiratory syndrome coronavirus 2 OX=2697049 GN=S PE=1 SV=1























In some embodiments, the SARS-CoV-2 polypeptide derives from the papain-like protease. In some embodiments, not mentioned as such in the claims of this patent, but still not inconsistent with the claimed subject-matter, the SARS-CoV-2 polypeptide has an amino acid sequence having at least 90% of identity with the amino acid sequence as set forth in SEQ ID NO:3. In some embodiments, not mentioned as such in the claims of this patent, but still not inconsistent with the claimed subject-matter, the c SARS-CoV-2 polypeptide has an amino acid sequence having at least 90% of identity with the amino acid sequence that ranges from the amino acid residue at position 746 to the amino acid residue at position 1060 in SEQ ID NO:3.

SEQ ID NO:3 >sp|P0DTD1|R1AB_SARS2 Replicase polyprotein 1ab OS=Severe
acute respiratory syndrome coronavirus 2 OX=2697049 GN=rep PE=1 SV=1


























































































































In the present text, a first amino acid sequence having at least 90% of identity with a second amino acid sequence means that the first sequence has 90; 91; 92; 93; 94; 95; 96; 97; 98; 99 or 100% of identity with the second amino acid sequence. Sequence identity is frequently measured in terms of percentage identity (or similarity or homology); the higher the percentage, the more similar are the two sequences. Methods of alignment of sequences for comparison are well known in the art. Various programs and alignment algorithms are described in: Smith and Waterman, Adv. Appl. Math., 2:482, 1981; Needleman and Wunsch, J. Mol. Biol., 48:443, 1970; Pearson and Lipman, Proc. Natl. Acad. Sci. U.S.A., 85:2444, 1988; Higgins and Sharp, Gene, 73:237-244, 1988; Higgins and Sharp, CABIOS, 5:151-153, 1989; Corpet et al. Nuc. Acids Res., 16:10881-10890, 1988; Huang et al., Comp. Appls Biosci., 8:155-165, 1992; and Pearson et al., Meth. Mol. Biol., 24:307-31, 1994). Altschul et al., Nat. Genet., 6:119-129, 1994, presents a detailed consideration of sequence alignment methods and homology calculations. By way of example, the alignment tools ALIGN (Myers and Miller, CABIOS 4:11-17, 1989) or LFASTA (Pearson and Lipman, 1988) may be used to perform sequence comparisons (Internet Program^{®} 1996, W. R. Pearson and the University of Virginia, fasta20u63 version 2.0u63, release date December 1996). ALIGN compares entire sequences against one another, while LFASTA compares regions of local similarity. These alignment tools and their respective tutorials are available on the Internet at the NCSA Website, for instance. Alternatively, for comparisons of amino acid sequences of greater than about 30 amino acids, the Blast 2 sequences function can be employed using the default BLOSUM62 matrix set to default parameters, (gap existence cost of 11, and a per residue gap cost of 1). When aligning short peptides (fewer than around 30 amino acids), the alignment should be performed using the Blast 2 sequences function, employing the PAM30 matrix set to default parameters (open gap 9, extension gap 1 penalties). The BLAST sequence comparison system is available, for instance, from the NCBI web site; see also Altschul et al., J. Mol. Biol., 215:403-410, 1990; Gish. & States, Nature Genet., 3:266-272, 1993; Madden et al. Meth. Enzymol., 266:131-141, 1996; Altschul et al., Nucleic Acids Res., 25:3389-3402, 1997; and Zhang & Madden, Genome Res., 7:649-656, 1997.

In some embodiments, the SARS-CoV-2 polypeptide is attached to the surface of the particle by any conventional method well known in the art, such as described in Hermanson, Greg T. Bioconjugate techniques. Academic press, 2013. In some embodiments, 1-ethyl-3-[3-dimethylaminopropyl] carbodiimide hydrochloride (EDC)- N-hydroxysulfosuccinimide (Sulfo NHS) reactions are used for conjugating the coronaviral polypeptides to the particles. In some embodiments, the particle is conjugated to an avidin moiety that can create an avidin-biotin complex with the biotinylated coronaviral polypeptides and the particles. Additional, appropriate cross-linking agents for use in the invention include a variety of agents that are capable of reacting with a functional group present on a surface of the particle. Reagents capable of such reactivity include homo- and hetero-bifunctional reagents, many of which are known in the art. Heterobifunctional reagents are preferred. A typical bifunctional cross-linking agent is N-succinimidyl(4-iodoacetyl) aminobenzoate (SIAB). However, other crosslinking agents, including, without limitation, dimaleimide, dithio-bis-nitrobenzoic acid (DTNB), N-succinimidyl-S-acetyl-thioacetate (SATA), N-succinimidyl-3-(2-pyridyldithio) propionate (SPDP), succinimidyl 4-(N-maleimidomethyl)cyclohexane-1-carboxylate (SMCC) and 6-hydrazinonicotimide (HYNIC) may also be used. For further examples of cross-linking reagents, see, e.g., S. S. Wong, "Chemistry of Protein Conjugation and Cross-Linking," CRC Press (1991), and G. T. Hermanson, "Bioconjugate Techniques," Academic Press (1995).

In some embodiments, the receptacle may be any solid container, for example a test tube, a microplate well or a reaction cuvette made of polypropylene.

In some embodiments, the elimination of the unbound reagents may be carried out by any technique known to those skilled in the art, such as e.g. washing by means of repeated centrifugation steps.

As used herein the term **"immunocomplex"** refers to the complex formed between the coronavirus-specific antibodies of the subject and their specific antigen, i.e. the coronaviral polypeptide that is conjugated to the particle.

The presence and amount of the immunocomplexes may be detected by methods known in the art, including label-based and label-free detection. In some embodiments, the method of the present invention includes use of a secondary antibody that is coupled to an indicator reagent comprising a signal generating compound. In some embodiments, the secondary antibody has specificity for a particular immunoglobulin. In some embodiments, the secondary antibody is an anti-human IgG antibody, including anti-IgG1, IgG2, IgG3 and IgG4 antibodies. In some embodiments, the secondary antibody is an anti-IgM antibody. In some embodiments, the secondary antibody is an anti-human IgA antibody, including anti-IgA1 and IgA2 antibodies. In some embodiments, the antibody having specificity for a particular type immunoglobulin is a rabbit or goat antibody. In some embodiments, the antibody of the present invention is a monoclonal antibody or a polyclonal antibody. Thus, in some embodiments, the method of the present invention is particularly suitable for detecting presence of IgM SARS-CoV-2-specific antibodies. The method of the present invention is particularly suitable for detecting presence of IgG SARS-CoV-2-specific antibodies. In some embodiments, the method of the present invention is also particularly suitable for detecting presence of IgA SARS-CoV-2-specific antibodies. In some embodiments, the method of the present invention is particularly suitable for detecting presence of IgG, IgM and IgA SARS-CoV-2-specific antibodies. Indicator reagents include chromogenic agents, catalysts such as enzyme conjugates, fluorescent compounds such as fluorescein and rhodamine, chemiluminescent compounds such as dioxetanes, acridiniums, phenanthridiniums, ruthenium, and luminol, radioactive elements, direct visual labels, as well as cofactors, inhibitors and magnetic particles. Examples of enzyme conjugates include alkaline phosphatase, horseradish peroxidase and beta-galactosidase. In some embodiments, the secondary antibody is conjugated to phycoerythrin.

Methods for detecting the particle identity codes, e.g., a fluorescent code, are known in the art and are described below. Examples of systems that read (detect or analyze) multiplex assay signals from Luminex beads include, e.g., the Luminex xMAP 100 and xMAP 200 instruments or the Bio-Plex 100 and Bio-Plex 200 from BioRad instruments. Another method for detecting and/or separating particle sets based on ID codes is flow cytometry. Methods of and instrumentation for flow cytometry are known in the art, and those that are known can be used in the practice of the present invention. Flow cytometry, in general, involves the passage of a suspension of the particles as a stream past a light beam and electro-optical sensors, in such a manner that only one particle at a time passes through the region. As each particle passes this region, the light beam is perturbed by the presence of the particle, and the resulting scattered and fluorescent light are detected. The optical signals are used by the instrumentation to identify the subgroup to which each particle belongs, along with the presence and amount of label, so that individual assay results are achieved. Descriptions of instrumentation and methods for flow cytometry are known in the art and include, e.g., McHugh, "Flow Microsphere Immunoassay for the Quantitative and Simultaneous Detection of Multiple Soluble Analytes," Methods in Cell Biology 42, Part B (Academic Press, 1994); McHugh et al., "Microsphere-Based Fluorescence Immunoassays Using Flow Cytometry Instrumentation," Clinical Flow Cytometry, Bauer, K. D., et al., eds. (Baltimore, Md., USA: Williams and Williams, 1993), pp. 535-544; Lindmo et al, "Immunometric Assay Using Mixtures of Two Particle Types of Different Affinity," J. Immunol. Meth. 126: 183-189 (1990); McHugh, "Flow Cytometry and the Application of Microsphere-Based Fluorescence Immunoassays," Immunochemica 5: 116 (1991); Horan et al., "Fluid Phase Particle Fluorescence Analysis: Rheumatoid Factor Specificity Evaluated by Laser Flow Cytophotometry," Immunoassays in the Clinical Laboratory, 185-189 (Liss 1979); Wilson et al, "A New Microsphere-Based Immunofluorescence Assay Using Flow Cytometry," J. Immunol. Meth. 107: 225-230 (1988); Fulwyler et al., "Flow Microsphere Immunoassay for the Quantitative and Simultaneous Detection of Multiple Soluble Analytes," Meth. Cell Biol. 33: 613-629 (1990); Coulter Electronics Inc., United Kingdom Patent No. 1,561,042 (published Feb. 13, 1980); and Steinkamp et al., Review of Scientific Instruments 44(9): 1301-1310 (1973).

Typically, the detecting step thus involved the use of detector. As used herein, the term "**detector**" is intended to mean a device or apparatus that converts the energy of contacted photons into an electrical response. For instance, the term can include an apparatus that produces an electric current in response to impinging photons such as in a photodiode or photomultiplier tube. A detector can also accumulate charge in response to impinging photons and can include, for example, a charge coupled device. In particular, the detector involves the use of a radiation source. As used herein, the term "**radiation source**" is intended to mean an origin or generator of propagated electromagnetic energy. The term can include any illumination sources including, for example, those producing electromagnetic radiation in the ultraviolet, visible and/or infrared regions of the spectrum. A radiation source can include, for example, a lamp such as an arc lamp or quartz halogen lamp, or a laser. As used herein, the term "**laser**" is intended to mean a source of radiation produced by light amplification by stimulated emission of radiation. The term can include, for example, an ion laser such as argon ion or krypton ion laser, helium neon laser, helium cadmium laser, dye laser such as a rhodamine 6G laser, YAG laser or diode laser. These and other lasers useful in the apparatus of the invention are known in the art as described, for example, in Shapiro, Practical Flow Cytometry, 3rd Ed. Wiley-Liss, New York (1995).

In some embodiments, the detector is a flow cytometer. As used herein, the term "**flow cytometer**" is intended to mean a device or apparatus having a means for aligning the particles in a sample stream and a detector aligned such that the particles individually enter a zone of detection. A sample stream can include any mobile phase that passes particles in single file including, for example, a fluid stream or fluid jet.

The method of the present invention involves the use of a multiplex technology. Multiplex technology is the collective term for a variety of techniques which can assess multiple immunoglobulin specificities simultaneously on small volumes of sample. The advantage of multiplex technology is that it is able to provide very rapid test times and very high throughput of samples.

In some embodiments, the groups of particles used in step a) of the above method differ from one another by their identity codes (e.g. fluorophores).

The method of the present invention is particularly suitable for simultaneously detecting immunoglobulins having specificity for the SARS-Cov-2 nucleoprotein (N), and the SARS-Cov-2 S 1 protein. Detection of the spike protein (S) (including a fragment thereof such as S2 or S2' fragments) and/or the Papain-like proteinase (PL-Pro) are not mentioned in the claims of this patent, but still not inconsistent with the claimed subject-matter. Thus, the method of present invention comprises the step of contacting the sample with at least 2groups of particles, each particles being conjugated to a particular SARS-Cov-2 antigen. According to the invention, the sample is contacted with a plurality of particles, wherein a polypeptide deriving from the N protein is attached to the surface of some particles and wherein a polypeptide deriving from the S 1 protein is attached to the surface of other particles. In some embodiments not mentioned in the claims of this patent, but still not inconsistent with the claimed subject-matter, the sample is also contacted with particles wherein a polypeptide deriving from the PL-Pro protein is attached to the surface.

In some embodiments, the method of the present invention involves an addressable laser bead immunoassay (ALBIA), which is commercially available on Luminex^{™}-based platforms. For instance, ALBIA is a semi-quantitative homogenous fluorescence-based microparticle immunoassay that can be used for the simultaneous detection of several immunogobulins (e.g. up to 10 immunoglobulins). Each antigen (i.e. N, S1, S2, S2' and/or PL-Pro SARS-CoV-2 polypeptides) is covalently coupled to a set of distinct uniform size colour-coded particles. The sample is then incubated with the particles in the single assay receptacle. The particles are then washed and then incubated with secondary anti-human IgG or IgM antibodies conjugated to a fluorescent label (e.g. phycoerythrin). After washing again, the particles are analysed on a system in which separate lasers identified antigen by bead colour and quantified the antibody by measuring the fluorescence of the fluorescent label. Said quantification thus indicated the level of the detected immunoglobulins.

The method of the present invention is particularly suitable for the diagnosis of coronavirus infection. As used herein, the term "**diagnosing**" or "**diagnosis**", as used herein, means identifying the coronavirus infection. In particular, the method of the present invention is particularly suitable for the diagnosis of Severe Acute Respiratory Syndrome (SARS). The method of the present invention is suitable for the diagnosis of COVID-19.

In particular, the method of the present invention is particularly suitable for discriminating subjects who are recently infected by the SARS-CoV-2 from those who are already immunized. The IgM immunoglobulins are the first antibodies to be produced in the body in response to an infection. IgM immunoglobulins are larger than IgG immunoglobulins and when present in high numbers, may indicate a recent or new active infection. In short, a positive IgM may be a sign of a current, or very recent, infection. On the contrary, presence of IgG SARS-CoV-2-specific antibodies indicates that the subject is immunized. Thus, the method by allowing the detection of IgM, IgG and/or IgA SARS-CoV-2 specific antibodies provides a quick, simple and accurate aided detection method for identifying infected patients, in particular COVID-19 patients.

The method of the present invention is also particularly suitable for the serologic follow-up and therapy control of SARS-CoV-2 infections, in particular COVID-19. In some embodiments, the method of the present invention is particularly useful for vaccine purposes.

As used herein, the term "**vaccine**" includes at least one antigen in a pharmaceutically acceptable vehicle useful for inducing an immune response in a host. Vaccine compositions can be administered in dosages and by techniques well known to those skilled in the medical or veterinary arts, taking into consideration such factors as the age, sex, weight, species and condition of the recipient animal, and the route of administration. The term "**vaccine candidate**" refers to a vaccine that is under development (e.g. preclinical testing or clinical trial).

In particular, the method can be carried out for determining whether a subject achieves a protection with a vaccine or a vaccine candidate comprising i) detecting by carrying out the method of the present invention the presence of SARS-CoV-2 specific antibodies (in particular IgG SARS-CoV-2 specific antibodies) ii) and concluding that the subject achieves a protection with the vaccine or vaccine candidate when the presence of SARS-CoV-2 specific antibodies is detected.

The method of the present invention is also suitable for determining whether a subject has to be vaccinated against SARS-CoV-2, said method comprising i) detecting by carrying out the method of the present invention the presence of SARS-CoV-2 specific antibodies (in particular IgG SARS-CoV-2 specific antibodies) ii) and concluding that the subject has to be vaccinated when the absence of SARS-CoV-2 specific antibodies is detected or conversely does not need to be vaccinated if the presence of SARS-CoV-2 specific antibodies is detected.

The method of the present invention also offers to the physicians a reliable tool for research purposes (e.g. selecting a candidate vaccine, assessing a therapy, studying the replication of the virus, or epidemiologic studies). The method of the present invention is also suitable for deciding measures of containment or decontainment for an individual, or for a group of individuals.

The method is also particularly suitable for deciding the most accurate clinical decisions. In particular, detection of IgG SARS-CoV-2-specific antibodies can render the subject eligible to immunosuppressive treatment. As used herein, the term "**immunosuppressive treatment**" refers to any substance capable of producing an immunosuppressive effect, e.g., the prevention or diminution of the immune response and in particular the prevention or diminution of the acute inflammatory responses. In some embodiments, the method of the present invention is particularly suitable for determining whether a subject is eligible for a therapy with a corticosteroid. As used, the term "**corticosteroids**" has its general meaning in the art and refers to class of active ingredients having a hydrogenated cyclopentoperhydrophenanthrene ring system endowed with an anti-inflammatory activity. Corticosteroid drugs typically include cortisone, cortisol, hydrocortisone (11β,17-dihydroxy, 21-(phosphonooxy)-pregn-4-ene, 3,20-dione disodium), dihydroxycortisone, dexamethasone (21-(acetyloxy)-9-fluoro-1β,17-dihydroxy-16α-m-ethylpregna-1,4-diene-3,20-dione), and highly derivatized steroid drugs such as beconase (beclomethasone dipropionate, which is 9-chloro-11-β, 17,21, trihydroxy-16β-methylpregna-1,4 diene-3,20-dione 17,21-dipropionate). Other examples of corticosteroids include flunisolide, prednisone, prednisolone, methylprednisolone, triamcinolone, deflazacort and betamethasone. corticosteroids, for example, cortisone, hydrocortisone, methylprednisolone, prednisone, prednisolone, betamethesone, beclomethasone dipropionate, budesonide, dexamethasone sodium phosphate, flunisolide, fluticasone propionate, triamcinolone acetonide, betamethasone, fluocinolone, fluocinonide, betamethasone dipropionate, betamethasone valerate, desonide, desoximetasone, fluocinolone, triamcinolone, triamcinolone acetonide, clobetasol propionate, and dexamethasone.

A further object of the present invention relates to a kit for performing the method of the present invention. The kit comprises one or more plurality of particles as above described and means for determining the immunocomplexes. Reagents for particular types of assays can also be provided in kits of the invention. Thus, the kits can include different groups of particles each identified by a specific identity, plates that comprises the single assay receptacles (e.g. a multiwell plate), and secondary antibodies as described above. In some embodiments, the kits comprise a device such as a detector as described above. The groups of particles, the plate, and the devices are useful for performing the immunoassay of the present invention. In addition, the kits can include various diluents and buffers, labelled conjugates or other agents for the detection of the specifically immunocomplexes, and other signal-generating reagents, such as enzyme substrates, cofactors and chromogens. Other components of a kit can easily be determined by one of skill in the art.

The invention will be further illustrated by the following figures and examples. However, these examples and figures should not be interpreted in any way as limiting the scope of the present invention.

### FIGURES:

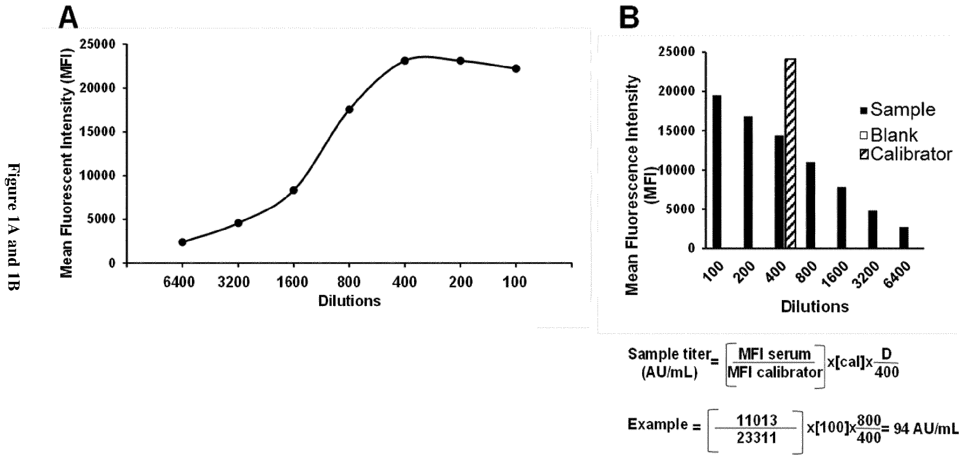

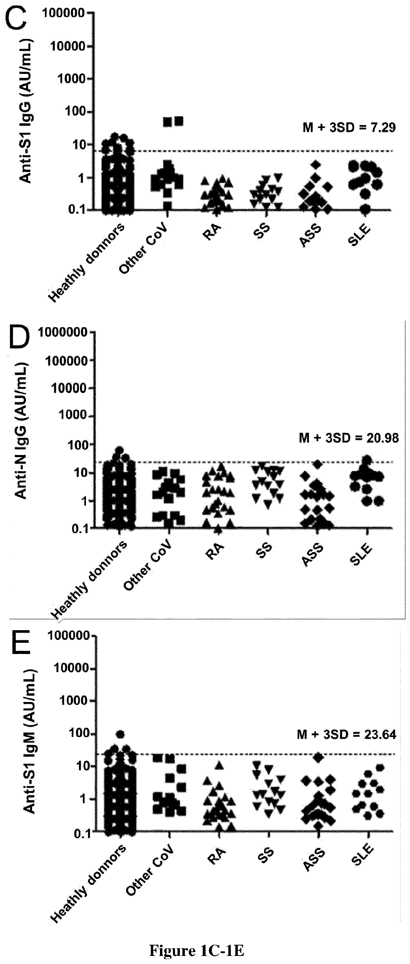

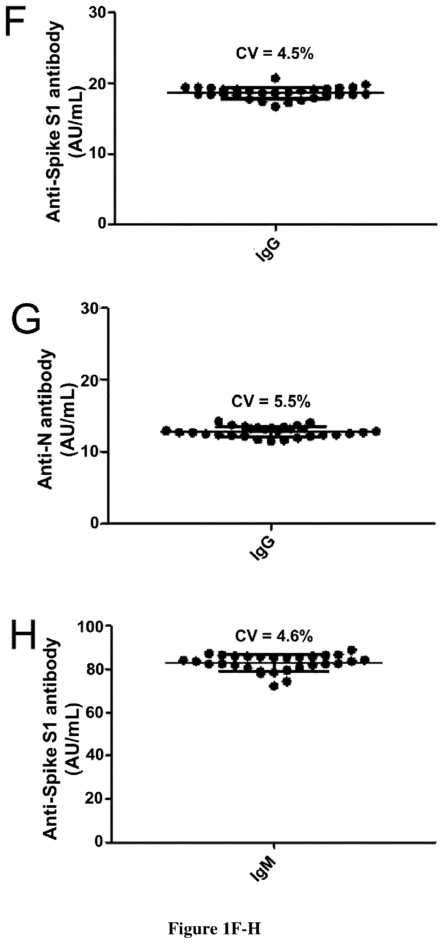

**Figure 1****. Detection, titration, and cross-reactivity of anti-SARS-CoV-2 Spike S1, nucleocapsid N protein IgG, and anti-SARS-CoV-2 Spike S1 IgM antibodies by ALBIA-IgG-S1/N and ALBIA-IgM-S1.**
   (A) A calibration curve was obtained after serial dilutions of the calibrator, i.e. one highly positive sample. A plateau of Mean Fluorescence Intensity (MFI) was reached for dilutions 1:400 or lower.
   (B) Calculation of antibody titer by reference to the MFI value of the calibrator (stripped bar) used at a 1/400 dilution in the assay and its level arbitrarily set to 100 arbitrary units (AU)/mL. The assay was first performed using a 1/100 screening dilution of the serum. In case the sample's MFI at 1/100 dilution is higher than 70% of the calibrator's MFI, further dilutions are performed and the first dilution yielding a MFI inferior to 70% of calibrator MFI is retained for calculation. An example is given: at 1/100 dilution, the MFI was higher than 70% of the calibrator's MFI (23,311 x 0.7=16,318), requiring a 1/800 dilution for computing the titer, i.e. 94 AU/mL anti-S 1 IgG level.
   (C-E) Specificity toward non-COVID-19 patients: (C) anti-Spike S1 and (C) anti-N IgG, IgM, and (E) anti-Spike S1 IgM antibody reactivity in patients with different conditions: PCR-confirmed infection with other CoV (17 sera from 13 patients; HKU1, n = 3; OC43, n = 11; NL63, n = 3). RA, rheumatoid arthritis; SS, Sjögren syndrome; ASS, antisynthetase syndrome; SLE, systemic lupus erythematosus.
   (F-H) Repeatability of (F) ALBIA-IgG-S1, (G) ALBIA-IgG-N, (H) ALBIA-IgM-S1. The assay was performed 30 times on the same sample, i.e. one serum from a PCR⁺Covid-19 patient used at a high working dilution of 1/100. Horizontal bars depict mean and standard deviation.

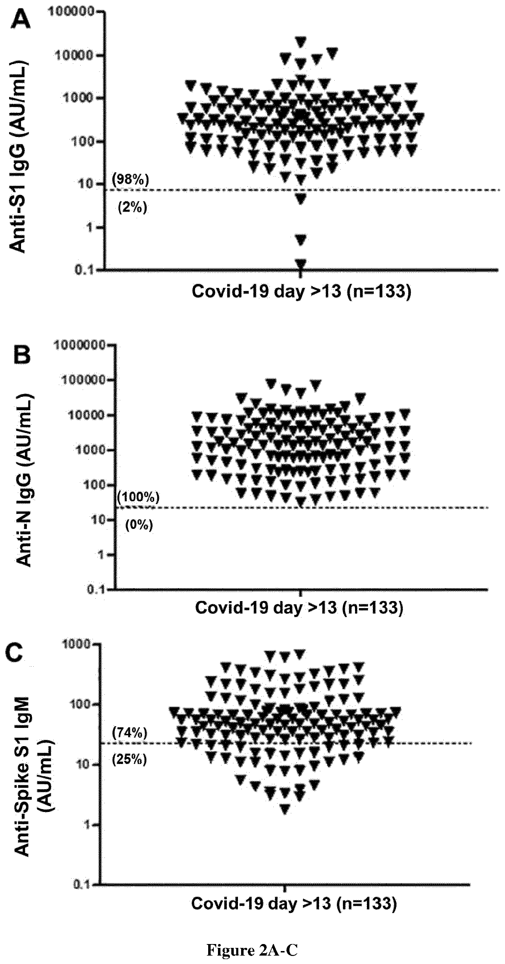

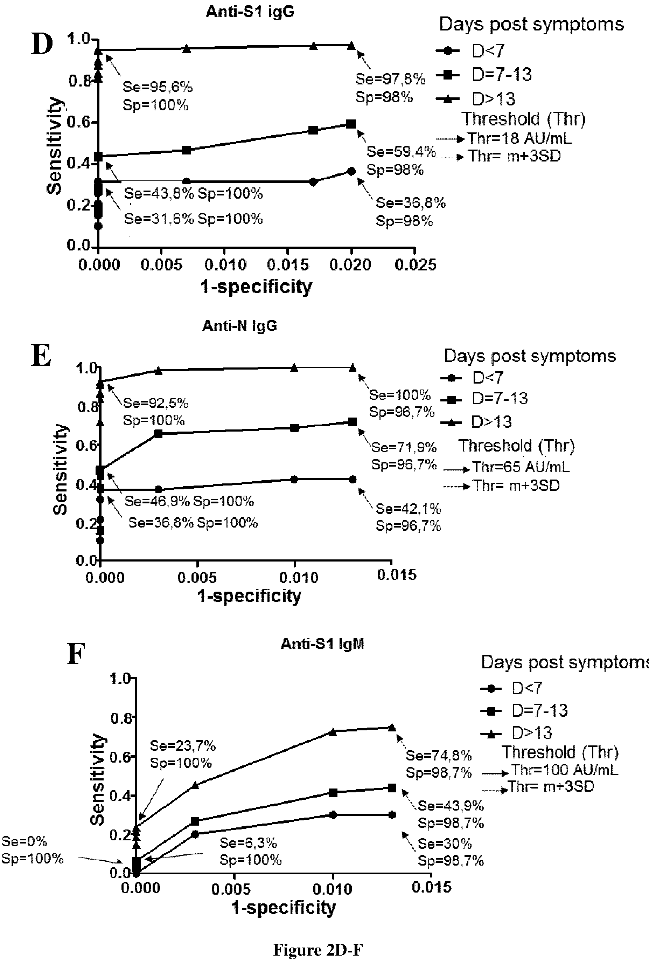

**Figure 2****. Antibody response to SARS-CoV-2 at day > 13 post infection.**
   (A) Anti-S1 IgG (median = 276 AU/mL), (B) Anti-N IgG (median = 1,434 AU/mL), (C) Anti-S1 IgM level (median = 48 AU/mL). Numbers in parenthesis indicate the percentages of data above and below the threshold. (D-F) Receiver Operating Characteristic (ROC) curve of ALBIA-IgG-S 1, ALBIA-IgG-N and ALBIA-S 1-IgM. The dotted line indicates the threshold value of 'mean C 3 standard deviations (M C 3SD)' of the control distribution. D, day post-symptoms. Se: Sensitivity and Sp: specificity.

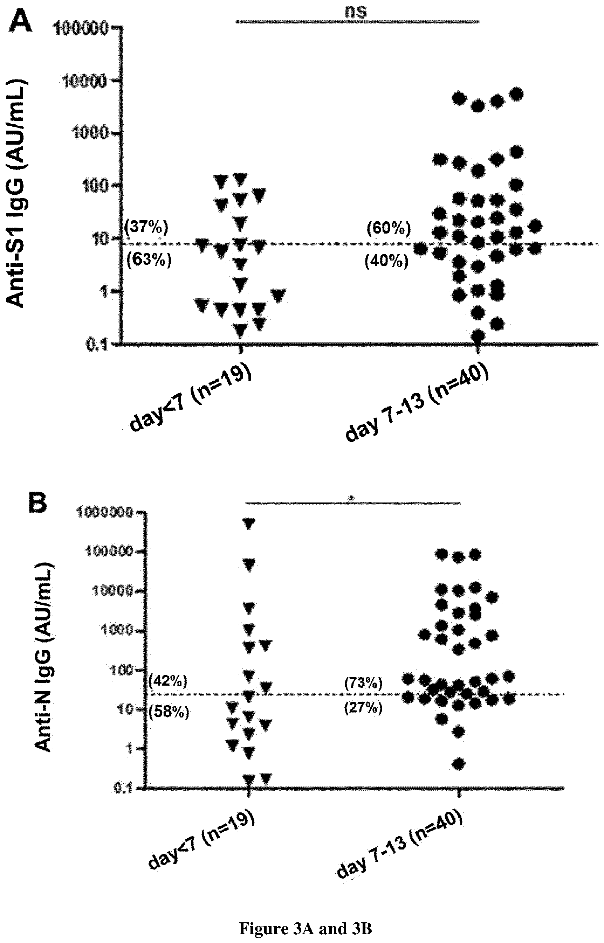

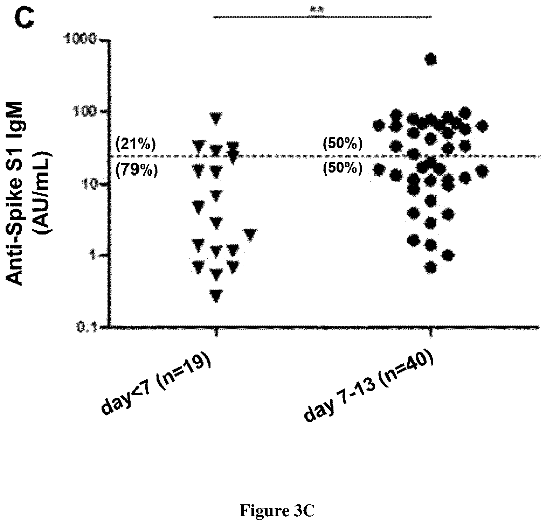

**Figure 3****. Levels of antibodies against SARS-CoV-2 at different times after symptom onset.**
   (A) Level of anti-S 1 IgG (median = 6 AU/mL and 13 AU/mL for day < 7and days 7-13, respectively). (B) Level of anti-N IgG (median = 11 AU/mL and 60 AU/mL for day < 7 and days 7-13, respectively). (C) Level of anti-S 1 IgM (median = 3 AU/mL and 23 AU/mL for day < 7 and days 7-13, respectively). Numbers in parenthesis indicate the percentages of data above and below the threshold. *P < 0.05, **P < 0.01 (Mann-Whitney test).

### EXAMPLE:

### Material and Methods

### Serum Samples

This is a retrospective study of serum samples from biorepositories of three French university hospitals authorized by the French Ministry of Research for the collection, analysis, storage, and reuse: Rouen University Hospital (authorization AC 2008-87), Limoges University Hospital (CRBioLim,authorization DC 2008-604), and Strasbourg University Hospital (authorization DC 2010-2222). All 192 sera analyzed, collected between March 23 and April 30, were from hospitalized or outpatients who had all been laboratory-confirmed positive for SARS-CoV-2 by RT-PCR of pharyngeal swab specimens. Of these 192 patients, 18 were hospitalized in the intensive care unit for a severe form of the disease.

Control sera were collected from 300 healthy blood donors (Etablissement Français du Sang, Lille, France), 13 patients with PCR-confirmed infections by other human coronaviruses (17 sera: HKU1, n = 3; OC43, n = 11; NL63, n = 3), and 70 patients with different inflammatory/autoimmune diseases according to established classification criteria: American College of Rheumatology revised criteria for systemic lupus erythematosus (SLE) (Tan et al., 1982) with anti-dsDNA aAbs (n = 12), American Rheumatism Association criteria for rheumatoid arthritis (RA) (Arnett et al., 1988) with anti-CCP Abs and/or rheumatoid factor (n = 23), revised European criteria for primary Sjögren syndrome (SS) (Vitali et al., 2002) with anti-SSA and/or anti-SSB aAbs (n = 14), and Troyanov criteria for antisynthetase syndrome (ASS) (Troyanov et al., 2005) (n = 21). All serum samples were stored at -80°C until use. Handling of serum samples was performed in a BSL-2 laboratory.

### Recombinant Proteins

Polyhistidine tagged recombinant Spike subunit 1 (S1, reference 40591-V08H) and nucleocapsid protein (N, reference 40588-V08B) were obtained from Sino Biologicals (Beijing, China). The identity and purity of these recombinant proteins were first determined by 4 to 10% gradient sodium dodecyl sulfate polyacrylamide gel electrophoresis (SDS-PAGE) under non-reducing conditions, followed by Coomassie blue staining. Western blot analysis was further performed by transfer of proteins separated by non-reducing SDS-PAGE to a nitrocellulose membrane followed by incubation with anti-6 x histidine monoclonal Ab (Sigma, St. Louis, MO,United States) and revelation with corresponding secondary Ab coupled to Alexa Fluor 680 (Invitrogen, Cergy Pontoise, France)

### Multiplex Addressable Laser Bead Immunoassay (ALBIA) for the Simultaneous Detection and Quantification of Anti-S1 and Anti-N IgG in COVID-19 Patients (ALBIA-IgG-S1/N)

To simultaneously detect anti-S1 and anti-N IgG from a single sample, we used two types of beads with a specific spectral signature. Color codes of S 1- and N-coupled beads were numbered 26 and 55 (Bio-Rad, Hercules, CA, United States), respectively; 10 mg of recombinant proteins was coupled to 1.25 x 106 fluorescent Bio-Plex^{R} COOH-microspheres (Bio-Rad) with the Bio-Plex^{R} amine coupling kit (Bio-Rad) according to manufacturer's protocol. After coupling, coated beads were either used immediately or stored at -20°C in the dark. Efficacy of coupling was validated using a commercial Ab recognizing the polyhistidine tag (Sigma), followed by a biotinylated goat anti-mouse IgG (Southern Biotech, Birmingham, AL,United States) secondary Ab. Revelation was then performed by incubation with 50 mL of streptavidin-R-PE (Qiagen, Venlo, Netherlands) for 10 min.

Immediately prior to their use, coated beads were vigorously agitated for 30 s. Then, a 10 mL volume of S1 and N protein coated beads (containing 1,250 beads) was added to 100 mL of serum from patients or controls [diluted in Dulbecco phosphate buffered saline (DPBS) plus 1% fetal bovine serum] in Bio-Plex Pro Flat bottom plates (Bio-Rad). Plates were incubated for 1 h at room temperature in the dark on a plate shaker at 650 rpm. Blank (no serum, secondary Ab only), negative controls (anti-S1 and anti-N Ab negative serum), and positive controls (human anti-S1 and anti-N Ab highly positive serum) were included in every assay. Beads were collected with a magnetic washer (Bio-Rad) and washed twice with 150 mL DPBS containing 0.1% Tween-20. Biotinylated mouse anti-human IgG-specific secondary Ab (Southern Biotech) was added at 1:2,000 dilution and incubated for 30 min at room temperature under shaking. After washing, beads were incubated with 50 mL of streptavidin-R-phycoerythrin at 1:1,000 dilution for 10 min. Finally, beads were resuspended in 100 mL of DPBS and mean fluorescence intensity (MFI) was determined on a Bio-Plex^{R} apparatus using the Bio-Plex^{R} Manager Software 4.0 (Bio-Rad) by experienced investigators (L.D., M.L.). A calibrator (i.e., a human serum from a PCR positive COVID-19 patient) with an MFI value reaching the plateau was included in each experiment.

Serum samples were initially assayed at 1:100 screening dilution. The calibrator was used at a dilution of 1:D' in the assay, and its level was arbitrarily set to 100 arbitrary units (AU)/mL. The Ab levels were determined at a dilution of 1:D, calculated using the following formula: ([MFI serum/MFI calibrator] x level of calibrator) x D/D'. When the MFI of a given serum sample at 1:100 dilution was higher than 70% of the calibrator MFI, further dilutions were performed. The first dilution yielding an MFI inferior to 70% of the calibrator MFI was retained for calculation of Ab titers (expressed in AU/mL).

For determination of repeatability, ALBIA was performed 30 times on the same positive serum. Coefficient of variation (CV) of the titer was determined as the ratio of the standard deviation (SD) to the mean.

Receiver operating characteristic (ROC) curves were computed by varying the threshold of positivity of the test, including one value consisting in the mean + 3 SD of negative controls.

### ALBIA for the Detection and Quantification of Anti-S1 IgM Abs (ALBIA-IgM-S1)

To detect anti-S1 IgM Abs, we used the same protocol as for ALBIA-IgG-S1/N except for the following modifications. Only S1-coupled beads were used. Anti-S1 IgM Abs were revealed using a biotinylated mouse anti-human IgM Ab (Southern Biotech) at 1:2,000 dilution for 30 min. Repeatability and Ab level were determined as described above.

### SARS-CoV-2 Ab Commercial Assays

Sera were tested using an N-based CLIA detecting IgG (Abbott SARS-CoV-2 IgG for Alinity automate), a Spike S1- and S2-based CLIA detecting IgG (Diasorin IgG for Liaison automate), and an S1-RBD-based anti-SARS-CoV-2 ELISA detecting total human Ig (Wantai SARS-CoV-2 Ab ELISA on SQ2 open platform), as per manufacturer's instructions.

### Statistical Analysis

Statistics were performed with Prism software (GraphPad, La Jolla, CA). Ab titers were compared using the non-parametric Mann-Whitney test. Concordance between the methods was analyzed using the K test. The interpretation of the K test depends on the calculated value of the coefficient K: discrepancy between the two tests (K < 0); very low agreement (0 < K < 0.2); low agreement (0.2 < K < 0.4); moderate agreement (0.4 < K < 0.6); good concordance (0.6 < K < 0.8); and excellent agreement (0.8 < K < 1).

### Results

### Validation of ALBIA-IgG-S1/N and ALBIA-IgM-S1

To allow quantitative analysis of anti-S1/N IgG or anti-S1 IgM in patients, we developed two ALBIAs (ALBIA-IgG-S1/N and ALBIA-IgM-S1, respectively). For this, we used as antigen polyhistidine-tagged recombinant Spike subunit 1 (S1) and nucleocapsid protein (N) of SARS-CoV-2. The identity and purity of these proteins were confirmed by Coomassie blue staining after SDS-PAGE, revealing a unique band (**data not shown**) that was specifically recognized by an anti-polyhistidine Ab in Western blot (**data not shown**).

S1 and N antigens were covalently coupled to fluorescent beads and used to determine the levels of anti-S1 and N IgG Abs, or anti-S1 IgM Abs. An example of the method used for calculating anti-S1 level is illustrated in **Figure 1**. A calibration curve was obtained after serial dilutions of a highly anti-S1-positive serum used as calibrator. A plateau of MFI was reached for dilution 1:400 (**Figure 1A**). At the screening dilution of 1:100, the sample used in this example showed a saturating signal (**Figure 1B**). A higher 1:800 dilution was retained to compute Ab level by reference to the calibrator whose level was arbitrarily set to 100 AU/mL. The same method of calculation was used for computing the levels of anti-N IgG and anti-S 1 IgM Ab.

ALBIA-IgG-S1/N was used to simultaneously investigate the presence of anti-S1 and anti-N IgG Ab. A threshold of positivity was calculated as the mean titer + 3 SD of the 300 negative control sera, which yielded values of 7.29 and 20.98 AU/mL for anti-S1 and anti-N IgG Ab, respectively (**Figures 1C, 1D**). For ALBIA-IgM-S 1, this threshold was 23.64 AU/mL (**Figure 1E**).

To evaluate potential cross-reactivity in our ALBIA between anti-SARS-CoV-2 Ab and other human coronaviruses, we tested 17 sera from 13 patients infected with HKU1, OC43, or NL63. An IgG reactivity to S1 but not N was found only once, in two sera from the same patient sampled at two different times post-infection with human coronavirus NL63 (Figures 1C-E). In addition, 70 patients with different inflammatory/autoimmune conditions leading to the production of rheumatoid factor or other auto-Abs, e.g., SLE, RA, SS, or ASS, were further tested. They all scored negative except for one lupus patient weakly positive for anti-N IgG (Figures 1C-E).

The diagnostic performance of the assay was determined using a collection of 133 sera from SARS-CoV-2-specific PCR-positive patients that were collected at least 14 days after first COVID-19 symptoms. ROC curve analysis of ALBIA-IgG-S1/N confirmed the accuracy of the aforementioned threshold value, i.e., mean + 3 SD. Indeed, sensitivity was 97.7% and specificity was 98.0% at a 7.29 AU/mL threshold for anti-S1 IgG (**2A, D**). For anti- N IgG Ab, sensitivity was 100% and specificity was 98.7% at a threshold of 20.98 AU/mL (**Figures 2E**). For ALBIA-IgM-S1, sensitivity and specificity were 74.8 and 98.7% at a threshold of 23.64 AU/mL (**Figures 2C****, F**).

### Repeatability of measures

Repeatability of the test was determined by calculating intra-assay variation for a given serum. CVs were 4.5 and 5.5 and 4.6% for anti-S1, anti-N IgG, and anti-S1 IgM, respectively (Figures 1F-H), indicating a good repeatability of this ALBIA.

### Frequency of Seropositivity During the Period of Seroconversion

Of the 192 samples from SARS-CoV-2 PCR-positive patients analyzed herein, 19 were collected up to day 7 after symptom onset, 40 between days 7 and 13, and 133 at day 14 or more after first symptoms. In the few asymptomatic patients of this series (n = 3), the time of positive SARS-CoV-2 PCR was used instead. The rate of positivity increased with time for all Abs tested (Figures 3A-C). The multiplex ALBIA-IgG-S1/N scored positive in 53% in the group day <7 (as compared to 37% for anti-S1 and 42% for anti-N IgG when considered separately; **Figures 3A****,B**), in 75% in the group days 7-13 (as compared to 60% for anti-S 1 and 73% for anti-N IgG; **Figures 3A****,B**) and 100% in the group day > 13 (as compared to 98% for anti-S1 and 100% for anti-N IgG; **Figures 2A****,B**). At the group level, an increase in Ab titers was observed with time (median value in group day <7, days 7-13 and day > 13: anti-S1 IgG, 6, 13, and 276 AU/mL; anti-N IgG, 11, 60, and 1,434 AU/mL; and anti-S1 IgM, 3, 23, and 48 AU/mL, respectively). All the differences between groups day > 13 and days 7-13, and between day > 13 and day <7, were statistically significant (Figures 2D-E). Anti-N IgG and anti-S1 IgM levels were also significantly higher in the group days 7-13 than in the group day < 7 (p < 0.05 and < 0.01, respectively; **Figures 3B****,C**), although the increase of anti-S 1 IgG levels was not statistically significant (p = 0.08; **Figure 3A**). When analyzed irrespectively of time of disease onset, 161 (84%) and 170 (89%) of the 192 patients of this series were positive for anti-S1 or anti-N IgG, respectively. Ab levels in seropositive patients were highly variable, ranging from 7.5 to 19,944 AU/mL, and from 24.74 to 491,992 AU/mL for anti-S1 or anti-N IgG, respectively. Using ALBIA-IgM-S1, 123 patients (64%) were positive, with titers ranging from 24.03 to 676 AU/mL. When combining the results of the three types of Ab (IgM, IgG S1, and N), the sensitivity reached 91%.

### Ab Levels in Patients Requiring Critical Care

Of this series, 18 patients had a severe form of disease requiring hospitalization in ICU. Anti-S1 (median = 511 AU/mL) and N (median = 2,930 AU/mL) IgG levels were significantly higher in these patients than in all other patients (anti-S1 IgG, median = 126 AU/mL; anti-N IgG, median = 696 AU/mL; p = 0.02 and 0.04, respectively). No statistically significant difference was found for anti-S 1 IgM (not shown).

### Comparison with Commercial EIA Assays

The performance of our novel assay was compared to that of different commercial assays on 76 available serum samples (10, 20, and 70 in groups day < 7, days 7-13, and day > 13, respectively). Global concordance of the multiplex ALBIA-IgGS1/N with Diasorin and Abbott assays was 91% and 93%, respectively, with K coefficients of 0.64 and 0.73 indicating a good concordance. Discordant tests were as follows: positivity of ALBIA when Diasorin was negative (n = 6/7), negativity of ALBIA when Diasorin was positive (n = 1/7), and positivity of ALBIA when Abbott was negative (n = 5/5). In addition, we analyzed the results of ALBIA according to the antigenic reactivity (anti-S or anti-N IgG). Concordance of ALBIA anti-S IgG with Diasorin was 93% with a coefficient K of 0.74 (good agreement). Concordance of ALBIA anti-N IgG with Abbott was 97% with a coefficient K of 0.91 (excellent agreement). Concordance of ALBIA IgG + IgM with the Wantai assay (detection of total Abs) was 95% with a K coefficient of 0.80 (excellent agreement).

### DISCUSSION

In this study, we report the high sensitivity and specificity of a new multiplex ALBIA for exploring the humoral immune response to SARS-CoV-2 subunit S1 (IgG and IgM) and nucleocapsid N protein (IgG). Since the emergence of COVID-19 at the end of 2019, efforts have been made to develop serological tests whose limitations have been widely outlined (Duong et al., 2020; Lai et al., 2020; Smithgall et al., 2020). Different health authorities or scientific organizations have issued recommendations on the performance that serological tests should have, i.e., a clinical specificity of at least 98% and a clinical sensitivity of 90% or more (Farnsworth and Anderson, 2020; Haute Autorité De Santé [HAS], 2020). Our multiplex ALBIA-IgG-S 1/N largely meets these criteria and confirms the excellent performance of bead immunoassays in accordance with a recent report (Ayoubaa et al., 2020). Our study further shows that the sensitivity of monoplex ALBIA-IgM-S1 remains around 75%, highlighting the fact that not all COVID-19 patients produce detectable levels of IgM (Guo et al., 2020; Liu et al., 2020).

The performance of current serological tests for COVID-19 has been judged perfectible in a large meta-analysis (Lisboa Bastos et al., 2020). Differences observed in sensitivity of such tests depend on the antigenic source used for each assay. Even if Abs directed against the viral S protein of SARS-CoV-2 are expected to appear earlier than those directed against the N protein (Liu et al., 2020), it has been shown that N-specific Abs were more sensitive than S-specific Abs for detecting early infection (Burbelo et al., 2020). Thus, multiplex assays offer several advantages. Allowing the simultaneous analysis of immune responses to different antigens, they increase the sensitivity of the test. Indeed, irrespectively of time of disease onset, the sensitivity of the multiplexed anti-S1 plus anti-N IgG assay (90%) was greater than the sensitivity of anti-S1 and anti-N IgG taken separately (84 and 89%, respectively). The sensitivity increases to 91% if the results of the anti-S1 IgM assay are also taken into account. Finally, combining several antigens in the same well reduces the cost and handling time of the assay.

Quantification of anti-S 1 IgM and IgG allows the study of the population dynamics of anti-S 1 IgG Ab response. Our results confirm that a 2-week delay is recommended for assaying IgG Ab in SARS-CoV-2-exposed patients in accordance with the literature (Huang, 2020). Also, the IgG levels of severely ill patients who required hospitalization in intensive care unit were significantly higher than those of patients with milder disease in accordance with a recent report (Long et al., 2020).

The diagnostic performance of ALBIA is equivalent to the best ELISAs or CLIAs reported in the literature (Bischof et al.,2020; Bryan et al., 2020; Kruttgen et al., 2020; Mahase, 2020;Montesinos et al., 2020; Traugott et al., 2020). Hence, we compared our novel assay with different commercially available CLIA or ELISA assays. Globally, our multiplex assay was more sensitive than the other assays tested. The best correlation was found with the Wantai ELISA, which detects total Abs against SARS-CoV-2 S1-RBD antigen, an assay already highlighted for its excellent performance (GeurtsvanKessel et al., 2020).

In conclusion, we have developed a highly sensitive and specific serological assay for exploring humoral immunity to SARS-CoV-2. This makes ALBIA a suitable tool for COVID-19 diagnosis and monitoring, epidemiological, or vaccination studies or for investigating the role of SARS-CoV-2 in non-typical forms of the disease (Hebert et al., 2020).

### REFERENCES:

Arnett, F. C.et al. (1988). The American Rheumatism Association 1987 revised criteria for the classification of rheumatoid arthritis. Arthr. Rheum 31, 315-324.

Ayoubaa, A., et al. (2020). Multiplex detection and dynamics of IgG antibodies to SARS-CoV2 and the highly pathogenic human coronaviruses SARSCoV and MERS-CoV. J. Clin. Virol. 129:104521. doi: 10.1016/j.jcv.2020.104521

Benveniste O et al. Correlation of anti-signal recognition particle autoantibody levels with creatine kinase activity in patients with necrotizing myopathy. Arthritis Rheum. 2011 Jul;63(7):1961-71.

Bischof, E., et al. (2020). Understanding COVID-19 new diagnostic guidelines - a message of reassurance from an internal medicine doctor in Shanghai. Swiss Med. Wkly. 150:w20216.

Bryan, A, et al. (2020). Performance Characteristics of the Abbott Architect SARS-CoV-2 IgG Assay and Seroprevalence in Boise, Idaho. J. Clin. Microbiol. 58:e00941-20.

Burbelo, P. D., et al. (2020). Sensitivity in detection of antibodies to nucleocapsid and spike proteins of severe acute respiratory syndrome coronavirus 2 in patients with Coronavirus Disease 2019. J. Infect. Dis. 222, 206-213. doi: 10.1093/infdis/jiaa273

Drouot L et al. Exploring necrotizing autoimmune myopathies with a novel immunoassay for anti-3-hydroxy-3-methyl-glutaryl-CoA reductase autoantibodies. Arthritis Res Ther. 2014 Feb 3;16(1):R39.

Duong, Y. T., Wright, G., and Justman, J. (2020). Antibody testing for coronavirus disease 2019: not ready for prime time. BMJ 370:m2655. doi: 10.1136/bmj.m2655

Farnsworth, C. W., and Anderson, N. W. (2020). SARS-CoV-2 serology: much hype, little data. Clin. Chem. 66, 875-877. doi: 10.1093/clinchem/hvaa107

GeurtsvanKessel, C. H., et al. (2020). An evaluation of COVID-19 serological assays informs future diagnostics and exposure assessment. Nat. Commun. 11:3436.

Guo L et al. Profiling Early Humoral Response to Diagnose Novel Coronavirus Disease (COVID-19). Clin Infect Dis. 2020 Mar 21. pii: ciaa310.

Hebert, V., et al. (2020). Lack of association between chilblains outbreak and SARS-CoV-2: histological and serological findings from a new immunoassay. J. Am. Acad. Dermatol. 83, 1434-1436. doi: 10.1016/j.jaad.2020.07.048

Huang, A. T. (2020). Systematic review of antibody mediated immunity to coronaviruses: antibody kinetics, correlates of protection, and association of antibody responses with severity of disease. medRxiv [preprint]. doi: 10.1101/2020.04.14.20065771

Kruttgen, A., et al. (2020). Comparison of four new commercial serologic assays for determination of SARS-CoV-2 IgG. J. Clin. Virol. 128:104394. doi: 10.1016/j.jcv.2020.104394

Lai, C. C., Wang, C. Y., Ko, W. C., and Hsueh, P. R. (2020). In vitro diagnostics of coronavirus disease 2019: technologies and application. JMII [Epub ahead of print]. doi: 10.1016/j.jmii.2020.05.016

Lisboa Bastos, et al. (2020). Diagnostic accuracy of serological tests for covid-19: systematic review and meta-analysis. BMJ 370:m2516. doi: 10.1136/bmj.m2516

Liu W et al. Evaluation of Nucleocapsid and Spike Pr 1 otein-based ELISAs for detecting antibodies against SARS-CoV-2. https://doi.org/10.1101/2020.03.16.20035014

Long, Q. X., et al. (2020). Antibody responses to SARS-CoV-2 in patients with COVID-19. Nat. Med. 26, 845-848.

Mahase, E. (2020). Covid-19: two antibody tests are "highly specific" but vary in sensitivity, evaluations find. BMJ 369:m2066. doi: 10.1136/bmj.m2066

Meyer, B., Drosten, C., and Muller, M. A. (2014). Serological assays for emerging coronaviruses: challenges and pitfalls. Virus Res. 194, 175-183. doi: 10.1016/j. virusres.2014.03.018

Montesinos, I., et al. (2020). Evaluation of two automated and three rapid lateral.flow immunoassays for the detection of anti-SARS-CoV-2 antibodies. J. Clin. Virol. 128:104413. doi: 10.1016/j.jcv.2020.104413

Okba N et al. SARS-CoV-2 specific antibody responses in COVID-19 patients. https://doi.org/10.1101/2020.03.18.20038059.

Smithgall, M. C., et al (2020). Types of assays for SARS-CoV-2 testing: a review. Lab. Med. 51, e59-e65.doi: 10.1093/labmed/lmaa039

Tan, E. M., et al. (1982). The 1982 revised criteria for the classification of systemic lupus erythematosus. Arthr. Rheum 25, 1271-1277. doi: 10.1002/art. 1780251101

Traugott, M., et al. (2020). Performance of SARS-CoV-2 antibody assays in different stages of the infection: comparison of commercial ELISA and rapid tests. J. Infect. Dis. 222,352-366.

Troyanov, Y., et al. (2005). Novel classification of idiopathic inflammatory myopathies based on overlap syndrome features and autoantibodies: analysis of 100 French Canadian patients. Medicine 84, 231-249. doi: 10.1097/01.md.0000173991.74008.b0

Vitali, C., et al. (2002). Classification criteria for Sjogren's syndrome: a revised version of the European criteria proposed by the American-European Consensus Group. Ann. Rheum. Dis. 61, 554-558. doi: 10.1136/ard.61.6.554

Xia N et al. Serological Test is an Efficient Supplement of RNA Detection for Confirmation of SARS-CoV-2 Infection. https://doi: 10.20944/preprints202003.0184.v1.

Xiang J et al. Evaluation of Enzyme-Linked Immunoassay and Colloidal Gold-Immunochromatographic Assay Kit for Detection of Novel Coronavirus (SARS-Cov-2) Causing an Outbreak of Pneumonia (COVID-19). BMJ doi:10.1101/2020.02.27.20028787

Xue-fei C et al. A Peptide-based Magnetic Chemiluminescence Enzyme Immunoassay for Serological Diagnosis of Corona Virus Disease 2019 (COVID-19). https://doi.org/10.1101/2020.02.22.20026617

## Claims

1. A method for detecting the presence of SARS-CoV-2-specific antibodies in a subject, comprising the steps of:
a) placing a sample which has been obtained from the subject in a single assay receptacle, in the presence of a plurality of particles belonging to at least two different groups, one of the groups being conjugated to a first coronaviral polypeptide and one other group being conjugated to a second coronaviral polypeptide,
b) incubating the mixture under conditions which allow the formation of immunocomplexes on each group of particles,
c) eliminating from the mixture of step b) the immunoglobulins which have not bound to the particles,
d) incubating the mixture of step c) with at least an anti-human IgG antibody that is coupled to a fluorescent label;
e) eliminating from the mixture of step d) the an anti-human IgG antibodies not bound to the immunocomplexes formed in step b), and
f) simultaneously detecting, by means of a detector capable of differentiating the at least two groups of particles mentioned above, the immunocomplexes formed in step d) on each particle, whereby the presence or absence of SARS-CoV-2-specific antibodies is revealed and wherein the fluorescence of the fluorescent label is measured to quantify the detected SARS-CoV-2-specific antibodies,
wherein one of the first and second coronaviral polypeptides derives from the SARS-CoV-2 nucleoprotein (N) and the other coronaviral polypeptide derives from the S1 subunit of the SARS-CoV-2 spike (S) protein.

2. The method of claim 1, wherein the sample has been obtained from the subject more than 13 days post infection by SARS-CoV-2.

3. The method of claim 1 and claim 2, wherein the groups of particles differ from one another by their identity codes.

4. Use of the method of any one of the preceding claims, for the diagnosis of SARS-CoV-2 infection.

5. A method for determining whether a subject achieves a protection with a vaccine or a vaccine candidate against SARS-CoV-2 comprising i) detecting by carrying out the method of any one of claims 1 to 3, the presence of SARS-CoV-2-specific antibodies, and ii) concluding that the subject achieves a protection with the vaccine or vaccine candidate when the presence of SARS-CoV-2-specific antibodies is detected.

6. A method for determining whether a subject has to be vaccinated against SARS-CoV-2, said method comprising i) detecting by carrying out the method of any one of claims 1 to 3, the presence of SARS-CoV-2-specific antibodies, and ii) concluding that the subject has to be vaccinated when the absence of SARS-CoV-2-specific antibodies is detected or conversely does not need to be vaccinated if the presence of SARS-CoV-2-specific antibodies is detected.

7. A kit for performing the method of any one of the preceding claims, comprising the particles and the secondary antibodies of any one of claims 1 to 3.

## Patentansprüche

1. Verfahren zum Feststellen des Vorhandenseins von SARS-CoV-2-spezifischen Antikörpern in einem Subjekt, umfassend die folgenden Schritte:
a) Einbringen einer Probe, die von dem Subjekt ermittelt wurde, in einen einzigen Assay-Behälter in Gegenwart einer Vielzahl von Partikeln, die zu mindestens zwei verschiedenen Gruppen gehören, wobei eine der Gruppen mit einem ersten coronaviralen Polypeptid konjugiert ist und eine andere Gruppe mit einem zweiten coronaviralen Polypeptid konjugiert ist,
b) Inkubieren der Mischung unter Bedingungen, die die Bildung von Immunkomplexen auf jeder Gruppe von Partikeln ermöglichen,
c) Eliminieren der Immunglobuline, die keine Bindung mit den Partikeln aufweisen, aus der Mischung von Schritt b),
d) Inkubieren der Mischung aus Schritt c) mit mindestens einem anti-humanen IgG-Antikörper, der an einen Fluoreszenzmarker gekoppelt ist;
e) Eliminieren der nicht an die in Schritt b) gebildeten Immunkomplexe gebundenen anti-humanen IgG-Antikörper aus der Mischung von Schritt d), und
f) gleichzeitiges Erfassen der in Schritt d) gebildeten Immunkomplexe auf jedem Partikel mittels eines Detektors, der in der Lage ist, die mindestens zwei oben aufgeführten Partikelgruppen zu unterscheiden, wodurch das Vorhandensein oder Fehlen von SARS-CoV-2-spezifischen Antikörpern aufgedeckt wird und wobei die Fluoreszenz der Fluoreszenzmarkierung gemessen wird, um die festgestellten SARS-CoV-2-spezifischen Antikörper zu quantifizieren, wobei eines der ersten und zweiten koronaviralen Polypeptide von dem SARS-CoV-2-Nukleoprotein (N) und das andere koronavirale Polypeptid von der S1-Untereinheit des SARS-CoV-2-Spitzenproteins (S) stammt.

2. Verfahren nach Anspruch 1, wobei die Probe von dem Subjekt mehr als 13 Tage nach der Infektion mit SARS-CoV-2 ermittelt wurde.

3. Verfahren nach Anspruch 1 und 2, wobei sich die Gruppen von Partikeln durch ihre Identitätscodes voneinander unterscheiden.

4. Verwendung des Verfahrens nach einem der vorstehenden Ansprüche zur Diagnose einer SARS-CoV- 2-Infektion.

5. Verfahren zum Bestimmen, ob ein Subjekt einen Schutz mit einem Impfstoff oder einem Impfstoffkandidaten gegen SARS-CoV-2 erreicht, umfassend i) Feststellen des Vorhandenseins von SARS-CoV-2-spezifischen Antikörpern durch Ausführen des Verfahrens nach einem der Ansprüche 1 bis 3, und ii) Schlussfolgern, dass das Subjekt einen Schutz mit dem Impfstoff oder Impfstoffkandidaten erreicht, wenn das Vorhandensein von SARS-CoV-2-spezifischen Antikörpern festgestellt wird.

6. Verfahren zum Bestimmen, ob ein Subjekt gegen SARS-CoV-2 geimpft werden muss, das Verfahren umfassend i) Feststellen des Vorhandenseins von SARS-CoV-2-spezifischen Antikörpern durch Ausführen des Verfahrens nach einem der Ansprüche 1 bis 3 und ii) Schlussfolgern, dass das Subjekt geimpft werden muss, wenn das Fehlen von SARS-CoV-2-spezifischen Antikörpern festgestellt wird, oder umgekehrt, dass es nicht geimpft werden muss, wenn das Vorhandensein von SARS-CoV-2-spezifischen Antikörpern festgestellt wird.

7. Kit zum Ausführen des Verfahrens nach einem der vorstehenden Ansprüche, umfassend die Partikel und die sekundären Antikörper nach einem der Ansprüche 1 bis 3.

## Revendications

1. Procédé de détection de la présence d'anticorps spécifiques du SARS-CoV-2 chez un sujet, comprenant les étapes suivantes :
a) le placement d'un échantillon qui a été obtenu à partir du sujet dans un unique réceptacle de dosage, en présence d'une pluralité de particules appartenant à au moins deux groupes différents, un des groupes étant conjugués à un premier polypeptide coronaviral et un autre groupe étant conjugué à un second polypeptide coronaviral,
b) la mise en incubation du mélange dans des conditions qui permettent la formation d'immunocomplexes sur chaque groupe de particules,
c) l'élimination, à partir du mélange de l'étape b), des immunoglobulines qui ne se sont pas liées aux particules,
d) la mise en incubation du mélange de l'étape c) avec au moins un anticorps anti-IgG humaine qui est couplé à un marqueur fluorescent ;
e) l'élimination, à partir du mélange de l'étape d), des anticorps anti-IgG humaine qui ne se sont pas liés aux immunocomplexes formés dans l'étape b), et
f) la détection simultanée, au moyen d'un détecteur capable de différencier les au moins deux groupes de particules mentionnés ci-dessus, des immunocomplexes formés dans l'étape d) sur chaque particule, grâce à quoi la présence ou l'absence d'anticorps spécifiques du SARS-CoV-2 est révélée et dans lequel la fluorescence du marqueur fluorescent est mesurée pour quantifier les anticorps spécifiques du SARS-CoV-2 détectés,
dans lequel un des premier et second polypeptides coronaviraux dérive de la nucléoprotéine (N) du SARS-CoV-2 et l'autre polypeptide coronaviral dérive de la sous-unité S1 de la protéine de spicule (S) du SARS-CoV-2.

2. Procédé selon la revendication 1, dans lequel l'échantillon a été obtenu à partir du sujet plus de 13 jours post-infection par le SARS-CoV-2.

3. Procédé selon la revendication 1 ou la revendication 2, dans lequel les groupes de particules diffèrent les uns des autres par leurs codes d'identité.

4. Utilisation du procédé selon l'une quelconque des revendications précédentes, pour le diagnostic d'une infection par le SARS-CoV-2.

5. Procédé pour déterminer si un sujet obtient une protection avec un vaccin ou un vaccin candidat contre le SARS-CoV-2 comprenant i) la détection, en mettant en oeuvre le procédé selon l'une quelconque des revendications 1 à 3, de la présence d'anticorps spécifiques du SARS-CoV-2, et ii) la conclusion que le sujet obtient une protection avec le vaccin ou le vaccin candidat quand la présence d'anticorps spécifiques du SARS-CoV-2 est détectée.

6. Procédé pour déterminer si un sujet doit être vacciné contre le SARS-CoV-2, ledit procédé comprenant i) la détection, en mettant en oeuvre le procédé selon l'une quelconque des revendications 1 à 3, de la présence d'anticorps spécifiques du SARS-CoV-2, et ii) la conclusion que le sujet doit être vacciné quand l'absence d'anticorps spécifiques du SARS-CoV-2 est détectée ou à l'inverse n'a pas besoin d'être vacciné si la présence d'anticorps spécifiques du SARS-CoV-2 est détectée.

7. Kit pour mettre en oeuvre le procédé selon l'une quelconque des revendications précédentes, comprend les particules et les anticorps secondaires selon l'une quelconque des revendications 1 à 3.
